# EUROPEAN PATENT APPLICATION

(11) **EP 4 640 833 A1**
(43) Date of publication of application: **29.10.2025**
(21) Application number: 23917335.4
(22) Date of filing: 28.12.2023
(51) Int. Cl.: C12N 15/39, C12N 15/62, A61K 39/275, A61K 39/285, A61P 31/20

(54) **MONKEYPOX VIRUS NUCLEIC ACID VACCINE AND USE THEREOF**

(30) Priority: 18.01.2023 CN 202310084275
(71) Applicant: Institute of Microbiology, Chinese Academy of Sciences, Beijing 100101 (CN)
(72) Inventor: GAO, Fu, Beijing 100101 (CN); WANG, Qihui, Beijing 100101 (CN); QI, Jianxun, Beijing 100101 (CN); DU, Pei, Beijing 100101 (CN); WANG, Han, Beijing 100101 (CN); KONG, Tianxiang, Beijing 100101 (CN)
(74) Representative: LLR
(86) International application number: PCT/CN2023/142779
(87) International publication number: WO 2024/152870

(57) **Abstract**

Provided are a polynucleotide encoding a chimeric or mixed antigen of poxvirus multiple immunogens, a related nucleic acid product thereof, and the use thereof in the preparation of a vaccine for preventing and/or treating poxvirus (in particular monkeypox virus) infection. The chimeric or mixed antigen of the poxvirus multiple immunogens encoded by the polynucleotide comprises two immunogens: a monkeypox virus A35R protein or an antigenic fragment thereof (or an appropriate variant thereof) and a monkeypox virus M1R protein or an antigenic fragment thereof (or an appropriate variant thereof). The immunogen components of the chimeric or mixed nucleic acid vaccine based on the polynucleotide are clear, and the chimeric or mixed nucleic acid vaccine can efficiently stimulate specific immune responses (for example, generating a protective antibody) against poxvirus (in particular monkeypox virus), can be used for preventing and/or treating poxvirus (in particular monkeypox virus), and has high clinical application prospects.

## Description

### Cross-Reference to Related Application

The present application claims priority to Chinese patent application No. 202310084275.7 filed on January 18, 2023 and entitled "MONKEYPOX VIRUS NUCLEIC ACID VACCINE AND USE THEREOF", which is incorporated herein by reference in its entirety.

### Technical Field

The present application relates to the field of biomedicine, in particular to a nucleic acid vaccine against monkeypox virus and use thereof.

### Background Art

Poxviruses, represented by monkeypox virus, are a class of nucleocytoplasmic large DNA viruses. Their viral genomes are about 130-375 kbp in size, and can encode up to 200 viral proteins. In addition to the fact that the encoded viral proteins are complex, viral particles of poxviruses are also relatively complex, and have two types of infectious viral particles with different morphologies, called intracellular mature virus (IMV) and extracellular envelope virus (EEV), respectively. Among them, IMV, which has an envelope, has higher stability than EEV and is mainly involved in the virus transmission between hosts. EEV, which has a special outer membrane structure, is mainly involved in the spread of the virus within the host. Because IMV and EEV have different membrane structures, membrane components, and cell infection mechanisms, their surface neutralizing antigens are also completely different.

Monkeypox virus (MPXV), which is a poxvirus and is highly homologous to the smallpox virus, has been spreading locally in central and western Africa for a long time and has evolved multiple strains since its discovery in 1958. However, in 2022, MPXV showed a global epidemic trend. By July 23, it had spread to 75 countries and infected more than 15,000 people, and was thus declared a "public health emergency of international concern" by the World Health Organization. Although current vaccines provide defense against the monkeypox virus, it is worth noting that recent findings show that MPXV has a trend of increasing mutation frequency, and there is the possibility of producing escape mutant strains.

There are only two vaccines worldwide that can be used for preventing monkeypox virus infection, namely JYNNEOS^{™} vaccine produced by Ankara-Bavarian Nordic (also known as Imvamune or Imvanex) and ACAM2000^{®} produced by Sanofi Pasteur. The two vaccines are attenuated live vaccines originally developed for preventing smallpox. ACAM2000^{®}, a second-generation vaccine, has replication capacity in the human body and thus brings risks such as encephalitis, myocarditis, and progressive vaccinia infection after vaccination, and is also unsuitable for vaccination of young children, pregnant women, and individuals with low or impaired immune function. By contrast, JYNNEOS^{™}, a third-generation vaccine, exhibits improved safety compared to the first-generation and second-generation vaccines since it cannot be replicated in the human body, but its immune efficacy is moderately reduced.

Live virus vaccines have the following common shortcomings: (1) the vaccines are derived from live viruses, and obtained by attenuated or weakened culture, and thereby they completely retain the antigens of the viruses, but there is the possibility of incomplete attenuation or restoration of virulence after mutation; (2) the vaccines contain all the antigens of the viruses, have complex components, and have a high possibility of causing side effects; (3) the proportion of effective immunogenic components in the vaccines is relatively low, resulting in weak immunogenicity even at the same dose of vaccination; (4) some viral components may inhibit host immunity, which will have a counterproductive effect and reduce the immunogenicity of the vaccine. Vaccines with defined components (e.g., mRNA vaccines/recombinant protein vaccines, etc.) can address the above problems. However, for complex viruses such as poxviruses, identifying key components to serve as immunogens is a major technical bottleneck.

The information disclosed in the Background section is intended solely to enhance understanding of the general background of the present application and shall not be construed as an acknowledgment or any form of suggestion that the information constitutes the prior art already known to those of ordinary skill in the art.

### Summary of the Invention

In order to overcome the above problems in the prior art, the present application provides a polynucleotide encoding a multi-immunogen chimeric or mixed poxvirus antigen, related nucleic acid products, and use thereof in the preparation of a vaccine for the prevention and/or treatment of poxvirus (especially monkeypox virus) infections; the multi-immunogen chimeric or mixed poxvirus antigen encoded by the polynucleotide contains two immunogens: a monkeypox virus A35R protein or its antigenic fragment (or their appropriate variants) and a monkeypox virus M1R protein or its antigenic fragment (or their appropriate variants). The chimeric or mixed nucleic acid vaccine based on the polynucleotide has clear immunogenic components and may efficiently elicit specific immune responses against poxviruses (especially the monkeypox virus), such as producing protective antibodies, which may be used for the prevention and/or treatment of poxvirus (especially the monkeypox virus) infections.

Specifically, the present application provides the following technical solutions.

In a first aspect, the present application provides a polynucleotide, which encodes a multi-immunogen chimeric or mixed poxvirus antigen, which antigen contains:
an immunogen I: a monkeypox virus A35R protein or its antigenic fragment, or an amino acid sequence having at least 90%, 92%, 95%, 96%, 97%, 98%, or 99% identity thereto and having the same or substantially the same immunogenicity therewith; and
an immunogen II: a monkeypox virus M1R protein or its antigenic fragment, or an amino acid sequence having at least 90%, 92%, 95%, 96%, 97%, 98%, or 99% identity thereto and having the same or substantially the same immunogenicity therewith.

In a feasible embodiment, the antigenic fragment of the A35R protein is an extracellular fragment of the protein or a part thereof, preferably a peptide fragment having an amino acid sequence as shown in SEQ ID NO: 1, or an amino acid sequence consisting of the amino acid sequence as shown in SEQ ID NO: 1 plus from 1 to 30 amino acids extending therefrom toward the N-terminus of the A35R protein, which sequence is preferably as shown in SEQ ID NO: 2;
and/or, the antigenic fragment of the M1R protein is an extracellular fragment of the protein or a part thereof, preferably a peptide fragment having an amino acid sequence as shown in SEQ ID NO: 3.

In some specific embodiments, the polynucleotide encodes a multi-immunogen mixed poxvirus antigen, which is a mixture of the immunogens I and II;
wherein, preferably, the immunogen I has an amino acid sequence as shown in SEQ ID NO: 1 or SEQ ID NO: 2, or an amino acid sequence that is derived from the amino acid sequence as shown in SEQ ID NO: 1 or SEQ ID NO: 2 by substitution, deletion, or addition of one or more amino acids, and exhibits the same or substantially the same immunogenicity therewith;
and/or, the immunogen II has an amino acid sequence as shown in SEQ ID NO: 3, or an amino acid sequence that is derived from the amino acid sequence as shown in SEQ ID NO: 3 by substitution, deletion, or addition of one or more amino acids, and exhibits the same or substantially the same immunogenicity therewith;
preferably, in the mixture, the mass ratio of the immunogens I and II is 1:1.

Further preferably, the polynucleotide is a group of polynucleotides, including:
I) a DNA molecule with a sequence as shown in SEQ ID NO: 4 or 5, and/or its corresponding mRNA molecule, which sequence is as shown in SEQ ID NO: 6 or 7 respectively; and
II) a DNA molecule with a sequence as shown in SEQ ID NO: 8, and/or its corresponding mRNA molecule, which sequence is as shown in SEQ ID NO: 9.

In other specific embodiments, the polynucleotide encodes a multi-immunogen chimeric poxvirus antigen, which is a single chain formed by one or more immunogens I and II in a series mode; preferably, the chimeric antigen has a structure as shown in Formula (I):

Al-C₁-M1-C₂-A2-C₃-M2 (I)

In the Formula (I):
A1 represents the monkeypox virus A35R protein or its antigenic fragment I, or an amino acid sequence having at least 90%, 92%, 95%, 96%, 97%, 98%, or 99% identity thereto and having the same or substantially the same immunogenicity therewith,
A2 represents the monkeypox virus A35R protein or its antigenic fragment II, or an amino acid sequence having at least 90%, 92%, 95%, 96%, 97%, 98%, or 99% identity thereto and having the same or substantially the same immunogenicity therewith,
M1 represents the monkeypox virus M1R protein or its antigenic fragment I, or an amino acid sequence having at least 90%, 92%, 95%, 96%, 97%, 98%, or 99% identity thereto and having the same or substantially the same immunogenicity therewith,
M2 represents the monkeypox virus M1R protein or its antigenic fragment II, or an amino acid sequence having at least 90%, 92%, 95%, 96%, 97%, 98%, or 99% identity thereto and having the same or substantially the same immunogenicity therewith;
C₁, C₂, and C₃ are each independently null, or a linker sequence (GGGGS)n, wherein n is any integer between 1 and 10; and,
wherein,
A1 and A2 are the same or different,
M1 and M2 are the same or different.
In a feasible embodiment, the antigenic fragment I or II of the A35R protein is an extracellular fragment of the protein or a part thereof;
and/or, the antigenic fragment I or II of the M1R protein is an extracellular fragment of the protein or a part thereof.

Preferably, A1 represents the amino acid sequence as shown in SEQ ID NO: 1, or an amino acid sequence that is derived from the amino acid sequence as shown in SEQ ID NO: 1 by substitution, deletion, or addition of one or more amino acids, and exhibits the same or substantially the same immunogenicity therewith;
and/or, A2 represents the amino acid sequence as shown in SEQ ID NO: 1, or an amino acid sequence consisting of the amino acid sequence as shown in SEQ ID NO: 1 plus from 1 to 30 amino acids extending therefrom toward the N-terminus of the A35R protein, which sequence is preferably as shown in SEQ ID NO: 2, or an amino acid sequence that is derived from any of the above amino acid sequences by substitution, deletion, or addition of one or more amino acids, and exhibits the same or substantially the same immunogenicity therewith;
and/or, M1 and M2 are the same and represent the amino acid sequence as shown in SEQ ID NO: 3, or an amino acid sequence that is derived from the amino acid sequence as shown in SEQ ID NO: 3 by substitution, deletion, or addition of one or more amino acids, and exhibits the same or substantially the same immunogenicity therewith;

In a preferred specific embodiment of the polynucleotide encoding the chimeric antigen shown in Formula (I), A1 represents the amino acid sequence as shown in SEQ ID NO: 1, A2 represents the amino acid sequence as shown in SEQ ID NO: 1 or SEQ ID NO: 2, M1 and M2 are the same and represent the amino acid sequence as shown in SEQ ID NO: 3;
preferably, C1, C2, and C3 are all null;
further preferably, the chimeric antigen contains an amino acid sequence as shown in SEQ ID NO: 10 or 11;
optionally, the chimeric antigen is of a single-chain dimer structure.

In a further preferred specific embodiment, the polynucleotide encoding the chimeric antigen shown in Formula (I) is a DNA molecule and/or its corresponding mRNA molecule;
preferably, the DNA molecule has a DNA sequence as shown in SEQ ID NO: 12 or 13, and its corresponding mRNA molecule has an mRNA sequence as shown in SEQ ID NO: 14 or 15.

In a second aspect, the present application provides a nucleic acid construct, which comprises the polynucleotide as described in the first aspect above and optionally, at least one expression regulatory element operatively linked to the polynucleotide.

In a third aspect, the present application provides an expression vector, which comprises the nucleic acid construct as described in the second aspect above.

In a fourth aspect, the present application provides a host cell, into which the polynucleotide as described in the first aspect above, the nucleic acid construct as described in the second aspect above, or the expression vector as described in the third aspect above is transformed or transfected.

In a fifth aspect, the present application provides use of the polynucleotide as described in the first aspect above, the nucleic acid construct as described in the second aspect above, the expression vector as described in the third aspect above, or the host cell as described in the fourth aspect above in the preparation of a medicament for preventing and/or treating poxvirus infections;
preferably, the poxvirus is selected from: monkeypox virus, smallpox virus, cowpox virus, and/or vaccinia virus;
optionally, the medicament is a vaccine, preferably an mRNA vaccine;
optionally, the vaccine is in a form of a nasal spray, an oral preparation, a suppository, or a parenteral preparation;
preferably, the nasal spray is selected from an aerosol, a spray, and a dry powder inhaler;
preferably, the oral preparation is selected from a tablet, a powder, a pill, granules, a soft/hard capsule, a film-coated agent, and a paste; further preferably, the tablet is a sublingual tablet; further preferably, the granules are fine granules; further preferably, the powder is a pulvis; and further preferably, the pill is a pilule;
preferably, the parenteral preparation is a transdermal preparation, an ointment, a plaster, a topical liquid preparation, or an injectable preparation; and further preferably, the injectable preparation is a preparation for infusion.

In a sixth aspect, the present application provides a nucleic acid vaccine or immunogenic composition, which comprises the polynucleotide as described in the first aspect above, the nucleic acid construct as described in the second aspect above, the expression vector as described in the third aspect above, or the host cell as described in the fourth aspect above, as well as a physiologically acceptable vehicle, adjuvant, excipient, carrier, and/or diluent.

In some preferred specific embodiments, the nucleic acid vaccine or immunogenic composition is a DNA vaccine against a poxvirus, which comprises:
(i) an eukaryotic expression vector; and
(ii) a DNA sequence of the polynucleotide as described in the first aspect above, which is constructed into the eukaryotic expression vector;
preferably, the eukaryotic expression vector is selected from pGX0001, pVAX1, pCAGGS, and pcDNA series vectors.

In other preferred specific embodiments, the nucleic acid vaccine or immunogenic composition is an mRNA vaccine against a poxvirus, which comprises:
(I) an mRNA sequence of the polynucleotide as described in the first aspect above; and
(II) lipid nanoparticles.

In other preferred specific embodiments, the nucleic acid vaccine or immunogenic composition is a poxvirus-viral vector vaccine, which comprises:
(1) a viral backbone vector; and
(2) a DNA sequence of the polynucleotide as described in the first aspect above, which is constructed into the viral backbone vector;

preferably, the viral backbone vector is selected from one or more of the following viral vectors: an adenovirus vector, a poxvirus vector, an influenza virus vector, and an adeno-associated virus vector. In a feasible embodiment, the nucleic acid vaccine or immunogenic composition is in a form of a nasal spray, an oral preparation, a suppository, or a parenteral preparation;
preferably, the nasal spray is selected from an aerosol, a spray, and a dry powder inhaler;
preferably, the oral preparation is selected from a tablet, a powder, a pill, granules, a soft/hard capsule, a film-coated agent, and a paste;
further preferably, the tablet is a sublingual tablet;
further preferably, the granules are fine granules;
further preferably, the powder is a pulvis;
further preferably, the pill is a pilule;
preferably, the parenteral preparation is a transdermal preparation, an ointment, a plaster, a topical liquid preparation, or an injectable preparation; and further preferably, the injectable preparation is a preparation for infusion.

In a seventh aspect, the present application provides a kit, which comprises the polynucleotide as described in the first aspect above, the nucleic acid construct as described in the second aspect above, the expression vector as described in the third aspect above, the host cell as described in the fourth aspect above, or the nucleic acid vaccine or immunogenic composition as described in the sixth aspect above.

In an eighth aspect, the present application provides a method for preventing and/or treating poxvirus infections, which comprises administering a prophylactically and/or therapeutically effective amount of the following substances to a subject in need thereof: the polynucleotide as described in the first aspect above, the nucleic acid construct as described in the second aspect above, the expression vector as described in the third aspect above, the host cell as described in the fourth aspect above, and/or the nucleic acid vaccine or immunogenic composition as described in the sixth aspect above.

The expression "prophylactically and/or therapeutically effective amount" may vary depending on the subject to be administered, the subject organ, the symptoms, the method of administration, etc., and may be determined by the physician's judgment, taking into account the type of the dosage form, the method of administration, the age and weight and symptoms of the patient, etc.

### Beneficial Effect

The present application provides a polynucleotide, which encodes a multi-immunogen chimeric or mixed poxvirus antigen. The chimeric or mixed antigen comprises a monkeypox virus A35R protein or its antigenic fragment (or their appropriate variants) and a monkeypox virus M1R protein or its antigenic fragment (or their appropriate variants). A chimeric or mixed nucleic acid vaccine based on the polynucleotide has the following advantages over prior art vaccines:
1) It addresses safety concerns of existing attenuated live virus vaccines; experimental verification shows that the poxvirus nucleic acid vaccine of the present application has good effectiveness, safety, and rapid responsiveness;
2) It has higher specificity against monkeypox virus; existing live virus vaccines are primarily developed based on vaccinia virus, which, while belonging to the same Poxviridae family as monkeypox virus, exhibits certain differences in neutralizing antigen sequences. Therefore, their protective efficacy against monkeypox virus remains uncertain. In contrast, the poxvirus nucleic acid vaccine of the present application is developed based on monkeypox virus epitopes, ensuring high specificity for the prevention and treatment of monkeypox virus;
3) Monkeypox virus contains various types of proteins, the vast majority of which are ineffective in eliciting antiviral immune responses, and therefore belong to ineffective components. In addition, some viral proteins even have immunosuppressive effects. Existing live virus vaccines may not remove the above ineffective and harmful components, leading to potential risks and uncertainties. In contrast, the poxvirus nucleic acid vaccine of the present application excludes components that are ineffective or harmful for eliciting effective immune responses in the host from the original immunogens, avoiding the inherent safety issues of attenuated vaccines. Moreover, experimental data confirm that the chimeric or mixed nucleic acid vaccine of the present application, which contains only the two immunogens mentioned above, may demonstrate complete protective effects in mouse models.

### Brief Description of the Drawings

One or more examples are exemplified by the pictures in the accompanying drawings that correspond thereto and are not intended to be limiting of the embodiments. As used herein, the word "exemplary" means "serving as an instance or an example, or illustrative". Any example described herein as "exemplary" is not necessarily to be construed as superior or better than other examples.

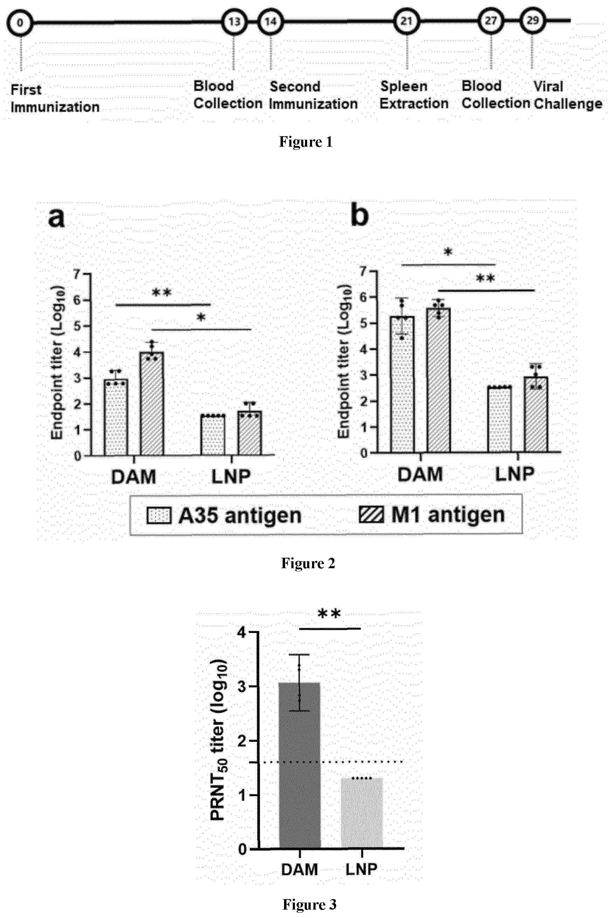

Figure 1 is a schematic diagram of a vaccine immunization and sampling procedure for mice adopted in Example 2 of the present application.
Figure 2 shows the detection results of the titers of specific binding antibodies against A35R and M1R antigens in the serum of immunized mice as detected in Example 3 of the present application, wherein Figure 2a shows the titers of binding antibodies against the A35R and M1R antigens in the serum of immunized mice collected on Day 13, and Figure 2b shows the titers of binding antibodies against the A35R and M1R antigens in the serum of immunized mice collected on Day 27.
Figure 3 shows the plaque neutralization results of the serum of immunized mice as detected in Example 4 of the present application.

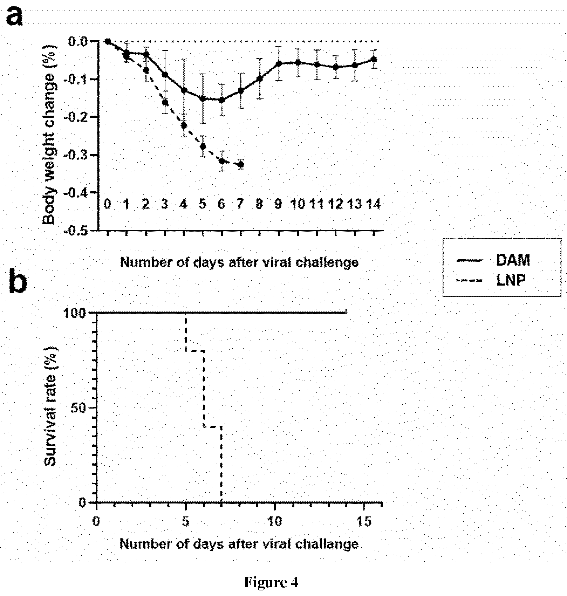

Figure 4 shows the protective effect of the chimeric mRNA vaccine DAM of the present application on BALB/c mice subjected to intranasal challenge with VACV-WR virus as detected in Example 5 of the present application, wherein the abscissa represents the number of days after the challenge, and the ordinate represents the weight change percentage (a) and the survival rate (b) of the mice.

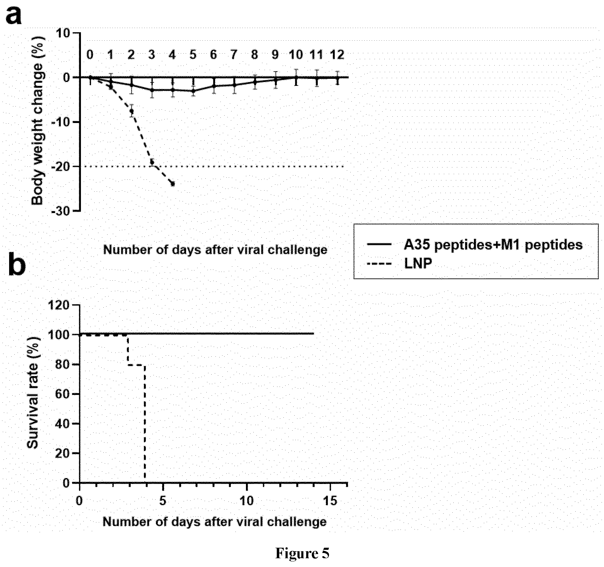

Figure 5 shows the protective effect of the mixed mRNA vaccine "A/M" of the present application on the BALB/c mice subjected to intranasal challenge with VACV-WR virus as detected in Example 5 of the present application, wherein the abscissa represents the number of days after the challenge, and the ordinate represents the weight change percentage (a) and the survival rate (b) of the mice.

### Detailed Description of the Invention

In order to make the objective, technical solutions and advantages of the present application clearer, the technical solutions in the examples of the present application will be described clearly and completely, obviously, the described examples are some of the examples of the present application, but not all of them. Based on the examples of the present application, all other examples obtained by those of ordinary skill in the art without creative work are within the scope of the present application.

In addition, in order to better explain the present application, a lot of specific details are given in the following particular embodiments. It will be understood by those skilled in the art that the present application may be practiced without certain specific details. In some examples, materials, elements, methods, means, etc., well known to those skilled in the art, are not described in detail so as to highlight the spirit of the present application.

Throughout the specification and claims, the term "comprise" or variations thereof, such as "comprising" or "comprised", will be understood to include the stated components and not to exclude other elements or other components, unless expressly indicated otherwise.

### Example 1: Antigen design, in vitro preparation, and lipid nanoparticle encapsulation of multi-immunogen chimeric or mixed mRNA vaccines against poxvirus

### Antigen design of mRNA vaccines:

As a representative example of the chimeric mRNA vaccine of the present application, in this example, an mRNA vaccine was constructed with the (A35R-M1R) dimer (hereinafter referred to as DAM) as the immunogen, which has an arrangement of A35R-M1R-A35R-M1R from the N-terminus to the C-terminus.

In the above DAM immunogen, the amino acid sequence of the first A35R peptide fragment starting from the N-terminus is shown in SEQ ID NO: 1, and the amino acid sequence of the second A35R peptide fragment is shown in SEQ ID NO: 2; the amino acid sequences of the two M1R peptide fragments are the same, as shown in SEQ ID NO: 3.

In addition, a mixed vaccine of A35R and M1R mRNA vaccines (hereinafter referred to as "A/M") was designed and prepared in this example. Specifically, mRNA vaccines of A35R and M1R single immunogens were prepared and encapsulated separately, and the two were mixed in a mass ratio of 1:1; wherein, the A35R and M1R single immunogens were the A35R peptide fragment as shown in SEQ ID NO: 1 and the M1R peptide fragment as shown in SEQ ID NO: 3 respectively.

In vitro preparation and lipid nanoparticle encapsulation:
1) In vitro transcription and capping of mRNA vaccine

In this example, the basic plasmid used for in vitro transcription of the mRNA vaccine was pUC57, provided by Nanjing Genscript Biotech Co., Ltd.

On the basic plasmid pUC57, DNA expression elements for the mRNA vaccine were introduced by conventional molecular biology means, including: (1) a T7 promoter, (2) the DNA coding region of the mRNA vaccine (the DNA coding sequence of the DAM chimeric mRNA vaccine is shown in SEQ ID NO: 13, and the DNA coding sequences of the "A35R" and "M1R" single mRNA vaccines are shown in SEQ ID NO: 4 and 8, respectively), (3) a 5'-end UTR sequence (as shown in SEQ ID NO: 16) upstream of the coding region, (4) the coding sequence (as shown in SEQ ID NO: 18) of a signal peptide (namely SP, as shown in SEQ ID NO: 17), and (5) a 3'-end UTR sequence (as shown in SEQ ID NO: 19) downstream of the coding region and a polyadenylic acid tail (Poly-A-tail). Firstly, the above in vitro transcription plasmid was digested with the restriction enzyme BamHI, and the plasmid was linearized; the linearized plasmid was purified by using a conventional DNA purification method to obtain a template for in vitro transcription; then, using the linearized plasmid as a template, in vitro transcription was performed with a T7 RNA in vitro transcription kit (E131-01A, Suzhou Novoprotein Scientific Co., Ltd.) to obtain in vitro transcribed mRNA; and finally, and the mRNA was purified by a lithium chloride precipitation method using a lithium chloride recovery kit (S125, Suzhou Novoprotein Scientific Co., Ltd.), to obtain purified in vitro transcribed mRNA.

Next, a Cap1 capping enzyme kit (M082-01B, Suzhou Novoprotein Scientific Co., Ltd.) was used to cap the above purified in vitro transcribed mRNA at the 5'-end with Cap1, to enable its translation in eukaryotic cells; afterwards, the mRNA was purified again by using the same lithium chloride precipitation method as described above to obtain purified mRNA with a 5'-end capping modification.

### 2) Lipid nanoparticle (LNP) encapsulation of mRNA

Cationic lipid, phosphatidylcholine, cholesterol, and PEG lipid were mixed at a ratio of 50:10:38.5:1.5, and then, using a Nanoassemblr Benchtop nanoliposome encapsulation instrument produced by Precision Nano Systems, the lipid mixture was mixed with and used to encapsulate the above 5'-end capped mRNAs (including "DAM" mRNA, as well as "A35R" or "M1R" single mRNA) separately. After the encapsulation, the buffer was replaced with phosphate buffer solution (PBS) via centrifugation or dialysis. After that, the mRNA encapsulation efficiency was determined using a Quan-iT Ribogreen RNA reagent kit from Thermo Fisher, and the encapsulation efficiency met the standards for mRNA vaccines.

In order to prepare the mixed mRNA vaccine "A/M" consisting of A35R and M1R single mRNA vaccines, the "A35R" and "M1R" single mRNA vaccines prepared and encapsulated by the above method were mixed at a mass ratio of 1:1.

### Example 2: Immunization of experimental animals and sample collection

In this example, animal experiments were performed using BALB/c female mice aged 6-8 weeks (purchased from Weitonglihua Experimental Animal Co., Ltd). The experimental groups were divided into mRNA vaccine immunization groups and a negative control group, wherein the mRNA vaccine immunization groups included the chimeric mRNA vaccine DAM and the mixed mRNA vaccine "A/M" immunization groups, and the negative control group was LNP immunization group. All mice in the mRNA vaccine immunization groups were immunized with one dose of the chimeric mRNA vaccine DAM prepared in Example 1, or the mixed mRNA vaccine "A/M" prepared in Example 1, on Day 0 and Day 14, respectively. Mice in the negative control group were injected with the same dose of empty LNP at the same time points. Vaccinations were administered via intramuscular injection at a dose of 7.5 µg mRNA vaccine or empty LNP per mouse at a time. Mouse serum samples were collected on Day 13 and Day 27, respectively, to determine the binding antibody titers and neutralization antibody titers in the serum of immunized mice, respectively. In addition, mouse spleen samples were collected on Day 21 to access T cell-mediated immune responses.

The mRNA vaccine immunization of mice and the sampling procedure were shown in Figure 1.

### Example 3: Detection of specific binding antibody titers in the serum of mice immunized with poxvirus chimeric mRNA vaccine

An ELISA plate was coated with monkeypox virus A35R and M1R antigen proteins (the amino acid sequences thereof were shown in SEQ ID NOs: 1 and 3, respectively), and then blocked with 5% skim milk for 1 hour. Then, sera collected from mice in each experimental group in Example 2 were incubated at 56°C for 30 minutes for inactivation. The inactivated serum samples were diluted in a three-fold gradient starting from 1:200 or 1:1000, then the dilution was added to each well, and the ELISA plate was subsequently incubated at 37 °C for 1 hour. Goat anti-mouse IgG-HRP antibody (purchased from EASYBio) was added to the plate as a secondary antibody and the plate was incubated at 37 °C for another 1 hour. Finally, 3,3',5,5'-tetramethylbenzidine (TMB) substrate was added for color development. After the color development was complete, the reaction was terminated with 2 M hydrochloric acid, and the absorbances at 450 nm and 630 nm were measured using a microplate reader (PerkinElmer). The absorbance value was calculated by subtracting the absorbance at 630 nm from that at 450 nm of the same well. The endpoint titer was defined as the corresponding dilution factor of serum when the absorbance produced by serum (the absorbance at 450 nm minus that at 630 nm as described above) was 2.1 times greater than the background value. An antibody titer below the detection limit was defined as one third of the detection limit.

The serum antibody titer detection results of the immunized mice were shown in Figure 2, where Figure 2a showed the antibody titer detection results of the serum collected on Day 13 of the immunization procedure, and Figure 2b showed the antibody titer detection results of the serum collected on Day 27 of the immunization procedure. As can be seen from Figure 2, the serum of mice upon first immunization (see Figure 2a) and second immunization (see Figure 2b) with the mRNA vaccine DAM both contained antibodies that specifically bind to the A35R and M1R antigen proteins, and the serum antibody titer after the second immunization increased by 1 to 2 orders of magnitude compared with that after the first immunization. These results demonstrated that the mRNA vaccine DAM can induce high levels of specific binding antibodies against the A35R and M1R antigen proteins.

### Example 4: Plaque neutralization assay with immunized mouse sera

Vaccinia virus (VACV), the model virus of the Orthopoxvirus genus, is an orthopoxvirus that can be handled in standard BSL-2 laboratories. In view of the minimal interspecies difference between VACV and other orthopoxvirus species, VACV is commonly used internationally to evaluate orthopoxvirus vaccines at the cellular and animal levels. Therefore, we used a mouse-adapted vaccinia virus strain Western Reserve (VACV-WR), which was frequently used internationally, to access the live virus neutralizing capacity of mRNA vaccines at the cellular level.

Specifically, Vero cells were plated in a 12-well plate, and one day later, the serum of mice immunized with the vaccine DAM as collected on Day 27 of the immunization procedure in Example 2 was serially diluted 10 times in a deep well plate with 2% FBS DMEM medium in a 2-fold gradient. An equal volume of 100 PFU of VACV virus was mixed with the diluted serum and incubated at 37°C for 1 hour. The cell culture supernatant was discarded and a serum-virus mixture was added to incubate at 37°C for 2 hours. The supernatant was discarded, the resultant was rinsed once with PBS, and the cells were overlaid with a 1:1 mixture of sodium carboxymethylcellulose and 2x DMEM, and cultured at 37°C for 48 hours. 4% paraformaldehyde was added to fix the cells at room temperature for 3 hours, and the mixture was discarded. Following staining with crystal violet for 120 minutes, plaques were counted using an ELISpot counter. Inhibition rate curve was plotted according to the reduction in plaque numbers compared to the positive control across different serum dilutions, and the IC50 (half-maximal inhibitory concentration) value of the curve was defined as the PRNT50 titer value of the serum sample.

The results were shown in Figure 3, which showed that the vaccine DAM can induce high levels of neutralizing antibodies against monkeypox virus, with a GMT (geometric mean titer) of 1160.

### Example 5: Challenge protection assay

Based on the inventors' preliminary experimental, all mice died 7 days after infection when challenged with 1.9 × 10⁵ PFU of VACV-WR, and therefore, this viral dose was used as the challenge dose for animal experiments in this example.

In this example, mice in the chimeric mRNA vaccine (DAM) immunization group, the mixed mRNA vaccine ("A/M") immunization group, and the LNP immunization group in Example 2 were subjected to VACV-WR intranasal challenge, respectively, on Day 29 after the first immunization, with the challenge dose being 1.9 × 105 PFU. By observing the survival rate and body weight changes of mice in the mRNA vaccine immunization groups and the negative control group, the protective efficacy of the mRNA vaccine DAM or "A/M" against live virus challenge in mice was evaluated.

The results were shown in Figures 4 and 5.

Figure 4 showed that the mRNA vaccine DAM according to the present application provided 100% protective efficacy against VACV-WR infection in mice, with an initial loss of body weight followed by rapid recovery. In contrast, the LNP-immunized mice experienced continuous body weight loss within 7 days until they all died.

Figure 5 showed that the mixed mRNA vaccine (i.e., "A/M") composed of A35R and M1R peptide fragments according to the present application also provided 100% protective efficacy against VACV-WR infection in mice, with an initial loss of body weight followed by rapid recovery. In contrast, the LNP-immunized mice experienced continuous body weight loss within 4 days until they all died.

To sum up, the multi-antigen chimeric or mixed nucleic acid vaccine of the present application can efficiently induce a specific immune response against a poxvirus (particularly the monkeypox virus), and thus is highly expected to be developed into a preventive and/or therapeutic vaccine product against a poxvirus (particularly the monkeypox virus), demonstrating great clinical application prospects.

Finally, it should be noted that the above embodiments are only used to illustrate, rather than to limit, the technical solutions of the present application. Although the present application has been described in detail with reference to the foregoing embodiments, it will be understood by one of ordinary skill in the art that the technical solutions described in the foregoing embodiments can still be modified or some technical features can be equivalently substituted. These modifications or substitutions do not make the essence of the corresponding technical solutions depart from the spirit and scope of the present application.

### Industrial Applicability

The chimeric or mixed nucleic acid vaccine against the monkeypox virus provided by the present application has clear immunogenic components and can efficiently induce specific immune responses against a poxvirus (especially the monkeypox virus), for example, producing protective antibodies, and thereby can be used for the prevention and/or treatment of poxviruses (especially the monkeypox virus), demonstrating great clinical application prospects.

### Sequences involved in the present application:

SEQ ID NO: 1 - the amino acid sequence of A35R antigenic fragment I (S89-T180) 
STTQYDHKESCNGLYYQGSCYILHSDYKSFEDAKANCAAESSTLPNKSDVLTTWLIDYVE
DTWGSDGNPITKTTSDYQDSDVSQEVRKYFCT
SEQ ID NO: 2 - the amino acid sequence of A35R antigenic fragment II (S63-T180) 
SANKAAITDSAVAVAAASSTHRKVVSSTTQYDHKESCNGLYYQGSCYILHSDYKSFEDAK
ANCAAESSTLPNKSDVLTTWLIDYVEDTWGSDGNPITKTTSDYQDSDVSQEVRKYFCT
SEQ ID NO: 3 - the amino acid sequence of M1R antigenic fragment 
AAASIQTTVNTLSERISSKLEQEANASAQTKCDIEIGNFYIRQNHGCNITVKNMCSADADA
QLDAVLSAATETYSGLTPEQKAYVPAMFTAALNIQTSVNTVVRDFENYVKQTCNSSAVVD
NKLKIQNVIIDECYGAPGSPTNLEFINTGSSKGNCAIKALMQLTTKATTQIAPRQVAG
SEQ ID NO: 4 - the DNA sequence encoding the A35R antigenic fragment I as shown in SEQ ID NO: 1 
AGCACCACCCAGTACGACCACAAAGAGAGCTGCAACGGCCTGTACTACCAAGGCTCT
TGTTACATCCTGCACAGCGACTATAAGAGCTTCGAGGATGCTAAGGCCAATTGCGCCGC
CGAGAGCAGCACACTGCCTAACAAGTCCGACGTGCTGACCACCTGGCTGATCGACTAC
GTGGAAGATACATGGGGAAGCGACGGCAACCCCATCACAAAAACCACAAGCGATTATC
AGGACAGCGACGTGTCTCAGGAGGTTAGAAAGTACTTTTGCACC
SEQ ID NO: 5 - the DNA sequence encoding the A35R antigenic fragment II as shown in SEQ ID NO: 2 TCCGCCAATAAGGCAGCCATCACCGACTCTGCCGTGGCAGTGGCAGCAGCCAGCTCCA 
CACACCGCAAGGTGGTGTCTAGCACCACACAGTACGATCACAAGGAATCCTGTAATGG
CCTGTACTATCAGGGCTCCTGTTACATCCTGCACTCTGACTATAAGAGCTTTGAGGATG
CCAAGGCCAACTGTGCCGCCGAGTCCTCTACCCTGCCAAATAAGAGCGACGTGCTGAC
CACATGGCTGATCGACTATGTGGAGGATACCTGGGGCTCTGATGGCAACCCCATCACAA
AGACCACATCTGATTACCAGGACAGCGACGTGAGCCAGGAGGTGCGGAAGTATTTCTG
CACC
SEQ ID NO: 6 - the mRNA sequence encoding the A35R antigenic fragment I as shown in SEQ ID NO: 1 
AGCACCACCCAGUACGACCACAAAGAGAGCUGCAACGGCCUGUACUACCAAGGCUC
UUGUUACAUCCUGCACAGCGACUAUAAGAGCUUCGAGGAUGCUAAGGCCAAUUGC
GCCGCCGAGAGCAGCACACUGCCUAACAAGUCCGACGUGCUGACCACCUGGCUGAU
CGACUACGUGGAAGAUACAUGGGGAAGCGACGGCAACCCCAUCACAAAAACCACAA
GCGAUUAUCAGGACAGCGACGUGUCUCAGGAGGUUAGAAAGUACUUUUGCACC
SEQ ID NO: 7 - the mRNA sequence encoding the A35R antigenic fragment II as shown in SEQ ID NO: 2 
UCCGCCAAUAAGGCAGCCAUCACCGACUCUGCCGUGGCAGUGGCAGCAGCCAGCUC
CACACACCGCAAGGUGGUGUCUAGCACCACACAGUACGAUCACAAGGAAUCCUGUA
AUGGCCUGUACUAUCAGGGCUCCUGUUACAUCCUGCACUCUGACUAUAAGAGCUU
UGAGGAUGCCAAGGCCAACUGUGCCGCCGAGUCCUCUACCCUGCCAAAUAAGAGCG
ACGUGCUGACCACAUGGCUGAUCGACUAUGUGGAGGAUACCUGGGGCUCUGAUGG
CAACCCCAUCACAAAGACCACAUCUGAUUACCAGGACAGCGACGUGAGCCAGGAGG
UGCGGAAGUAUUUCUGCACC
SEQ ID NO: 8 - the DNA sequence encoding the M1R antigenic fragment as shown in SEQ ID NO: 3 
GCCGCCGCCTCTATCCAGACCACAGTGAACACCCTGAGCGAGCGGATCAGCTCCAAGC
TGGAGCAGGAGGCAAACGCAAGCGCCCAGACAAAGTGCGACATCGAGATCGGCAATT
TCTACATCAGACAGAACCACGGCTGCAATATCACCGTGAAGAATATGTGCTCCGCCGA
 
CGCAGATGCACAGCTGGATGCCGTGCTGTCCGCCGCAACCGAGACATACTCTGGCCTG
ACCCCTGAGCAGAAGGCCTATGTGCCAGCCATGTTCACAGCCGCCCTGAACATCCAGA
CCAGCGTGAATACAGTGGTGCGGGACTTTGAGAACTACGTGAAGCAGACCTGCAATTC
TAGCGCCGTGGTGGACAACAAGCTGAAGATCCAGAATGTGATCATCGATGAGTGTTAT
GGAGCACCAGGCTCCCCTACCAACCTGGAGTTCATCAATACAGGCTCCTCTAAGGGCA
ACTGCGCCATCAAGGCCCTGATGCAGCTGACCACAAAGGCCACCACACAGATCGCAC
CTAGGCAGGTGGCAGGC
SEQ ID NO: 9 - the mRNA sequence encoding the M1R antigenic fragment as shown in SEQ ID NO: 3 
GCCGCCGCCUCUAUCCAGACCACAGUGAACACCCUGAGCGAGCGGAUCAGCUCCAA
GCUGGAGCAGGAGGCAAACGCAAGCGCCCAGACAAAGUGCGACAUCGAGAUCGGC
AAUUUCUACAUCAGACAGAACCACGGCUGCAAUAUCACCGUGAAGAAUAUGUGCU
CCGCCGACGCAGAUGCACAGCUGGAUGCCGUGCUGUCCGCCGCAACCGAGACAUAC
UCUGGCCUGACCCCUGAGCAGAAGGCCUAUGUGCCAGCCAUGUUCACAGCCGCCCU
GAACAUCCAGACCAGCGUGAAUACAGUGGUGCGGGACUUUGAGAACUACGUGAAG
CAGACCUGCAAUUCUAGCGCCGUGGUGGACAACAAGCUGAAGAUCCAGAAUGUGA
UCAUCGAUGAGUGUUAUGGAGCACCAGGCUCCCCUACCAACCUGGAGUUCAUCAAU
ACAGGCUCCUCUAAGGGCAACUGCGCCAUCAAGGCCCUGAUGCAGCUGACCACAAA
GGCCACCACACAGAUCGCACCUAGGCAGGUGGCAGGC
SEQ ID NO: 10 - the amino acid sequence of DAM (in which A1 and A2 are the same, both as shown in SEQ ID NO: 1) 
STTQYDHKESCNGLYYQGSCYILHSDYKSFEDAKANCAAESSTLPNKSDVLTTWLIDYVE
DTWGSDGNPITKTTSDYQDSDVSQEVRKYFCTAAASIQTTVNTLSERISSKLEQEANASAQ
TKCDIEIGNFYIRQNHGCNITVKNMCSADADAQLDAVLSAATETYSGLTPEQKAYVPAMF
TAALNIQTSVNTVVRDFENYVKQTCNSSAVVDNKLKIQNVIIDECYGAPGSPTNLEFINTG
SSKGNCAIKALMQLTTKATTQIAPRQVAGSTTQYDHKESCNGLYYQGSCYILHSDYKSFED
AKANCAAESSTLPNKSDVLTTWLIDYVEDTWGSDGNPITKTTSDYQDSDVSQEVRKYFCT
AAASIQTTVNTLSERISSKLEQEANASAQTKCDIEIGNFYIRQNHGCNITVKNMCSADADA
 
QLDAVLSAATETYSGLTPEQKAYVPAMFTAALNIQTSVNTVVRDFENYVKQTCNSSAVVD
NKLKIQNVIIDECYGAPGSPTNLEFINTGSSKGNCAIKALMQLTTKATTQIAPRQVAG
SEQ ID NO: 11 - the amino acid sequence of DAM (in which A1 and A2 are different) 
STTQYDHKESCNGLYYQGSCYILHSDYKSFEDAKANCAAESSTLPNKSDVLTTWLIDYVE
DTWGSDGNPITKTTSDYQDSDVSQEVRKYFCTAAASIQTTVNTLSERISSKLEQEANASAQ
TKCDIEIGNFYIRQNHGCNITVKNMCSADADAQLDAVLSAATETYSGLTPEQKAYVPAMF
TAALNIQTSVNTVVRDFENYVKQTCNSSAVVDNKLKIQNVIIDECYGAPGSPTNLEFINTG
SSKGNCAIKALMQLTTKATTQIAPRQVAGSANKAAITDSAVAVAAASSTHRKVVSSTTQYD
HKESCNGLYYQGSCYILHSDYKSFEDAKANCAAESSTLPNKSDVLTTWLIDYVEDTWGSD
GNPITKTTSDYQDSDVSQEVRKYFCTAAASIQTTVNTLSERISSKLEQEANASAQTKCDIEI
GNFYIRQNHGCNITVKNMCSADADAQLDAVLSAATETYSGLTPEQKAYVPAMFTAALNIQ
TSVNTVVRDFENYVKQTCNSSAVVDNKLKIQNVIIDECYGAPGSPTNLEFINTGSSKGNCA
IKALMQLTTKATTQIAPRQVAG
SEQ ID NO: 12 - the DNA sequence encoding DAM (in which A1 and A2 are the same, both as shown in SEQ ID NO: 1) 
AGCACCACCCAGTACGACCACAAAGAGAGCTGCAACGGCCTGTACTACCAAGGCTCT
TGTTACATCCTGCACAGCGACTATAAGAGCTTCGAGGATGCTAAGGCCAATTGCGCCGC
CGAGAGCAGCACACTGCCTAACAAGTCCGACGTGCTGACCACCTGGCTGATCGACTAC
GTGGAAGATACATGGGGAAGCGACGGCAACCCCATCACAAAAACCACAAGCGATTATC
AGGACAGCGACGTGTCTCAGGAGGTTAGAAAGTACTTTTGCACCGCCGCCGCCTCTAT
CCAGACCACAGTGAACACCCTGAGCGAGCGGATCAGCTCCAAGCTGGAGCAGGAGGC
AAACGCAAGCGCCCAGACAAAGTGCGACATCGAGATCGGCAATTTCTACATCAGACA
GAACCACGGCTGCAATATCACCGTGAAGAATATGTGCTCCGCCGACGCAGATGCACAG
CTGGATGCCGTGCTGTCCGCCGCAACCGAGACATACTCTGGCCTGACCCCTGAGCAGA
AGGCCTATGTGCCAGCCATGTTCACAGCCGCCCTGAACATCCAGACCAGCGTGAATAC
AGTGGTGCGGGACTTTGAGAACTACGTGAAGCAGACCTGCAATTCTAGCGCCGTGGTG
GACAACAAGCTGAAGATCCAGAATGTGATCATCGATGAGTGTTATGGAGCACCAGGCT
CCCCTACCAACCTGGAGTTCATCAATACAGGCTCCTCTAAGGGCAACTGCGCCATCAA
 
GGCCCTGATGCAGCTGACCACAAAGGCCACCACACAGATCGCACCTAGGCAGGTGGC
AGGCAGCACCACACAGTACGATCACAAGGAATCCTGTAATGGCCTGTACTATCAGGGC
TCCTGTTACATCCTGCACTCTGACTATAAGAGCTTTGAGGATGCCAAGGCCAACTGTGC
CGCCGAGTCCTCTACCCTGCCAAATAAGAGCGACGTGCTGACCACATGGCTGATCGAC
TATGTGGAGGATACCTGGGGCTCTGATGGCAACCCCATCACAAAGACCACATCTGATTA
CCAGGACAGCGACGTGAGCCAGGAGGTGCGGAAGTATTTCTGCACCGCTGCTGCTTCC
ATCCAGACCACAGTGAATACACTGAGCGAGAGAATCAGCTCCAAACTGGAGCAGGAG
GCCAACGCCTCTGCCCAGACCAAGTGTGACATCGAGATCGGCAACTTCTACATCAGGC
AGAATCATGGATGTAATATCACCGTGAAGAACATGTGCTCCGCCGATGCCGACGCCCAG
CTGGACGCCGTGCTGAGCGCCGCCACTGAGACATACTCCGGCCTGACCCCCGAACAG
AAAGCCTATGTGCCTGCTATGTTTACCGCCGCCCTGAACATTCAGACCAGCGTCAACAC
AGTGGTGCGCGATTTCGAAAATTATGTCAAACAGACTTGTAATTCTAGCGCCGTCGTCG
ATAACAAGCTGAAGATTCAGAATGTGATTATTGACGAATGCTACGGAGCACCAGGCTC
CCCAACCAACCTGGAGTTTATCAACACCGGCTCCTCTAAGGGCAATTGTGCCATCAAG
GCCCTGATGCAACTGACTACCAAAGCTACCACACAGATCGCACCAAGGCAGGTGGCA
GGA
SEQ ID NO: 13 - the DNA sequence encoding DAM (in which A1 and A2 are different) 
AGCACCACCCAGTACGACCACAAAGAGAGCTGCAACGGCCTGTACTACCAAGGCTCT
TGTTACATCCTGCACAGCGACTATAAGAGCTTCGAGGATGCTAAGGCCAATTGCGCCGC
CGAGAGCAGCACACTGCCTAACAAGTCCGACGTGCTGACCACCTGGCTGATCGACTAC
GTGGAAGATACATGGGGAAGCGACGGCAACCCCATCACAAAAACCACAAGCGATTATC
AGGACAGCGACGTGTCTCAGGAGGTTAGAAAGTACTTTTGCACCGCCGCCGCCTCTAT
CCAGACCACAGTGAACACCCTGAGCGAGCGGATCAGCTCCAAGCTGGAGCAGGAGGC
AAACGCAAGCGCCCAGACAAAGTGCGACATCGAGATCGGCAATTTCTACATCAGACA
GAACCACGGCTGCAATATCACCGTGAAGAATATGTGCTCCGCCGACGCAGATGCACAG
CTGGATGCCGTGCTGTCCGCCGCAACCGAGACATACTCTGGCCTGACCCCTGAGCAGA
AGGCCTATGTGCCAGCCATGTTCACAGCCGCCCTGAACATCCAGACCAGCGTGAATAC
AGTGGTGCGGGACTTTGAGAACTACGTGAAGCAGACCTGCAATTCTAGCGCCGTGGTG
GACAACAAGCTGAAGATCCAGAATGTGATCATCGATGAGTGTTATGGAGCACCAGGCT
 
CCCCTACCAACCTGGAGTTCATCAATACAGGCTCCTCTAAGGGCAACTGCGCCATCAA
GGCCCTGATGCAGCTGACCACAAAGGCCACCACACAGATCGCACCTAGGCAGGTGGC
AGGCTCCGCCAATAAGGCAGCCATCACCGACTCTGCCGTGGCAGTGGCAGCAGCCAG
CTCCACACACCGCAAGGTGGTGTCTAGCACCACACAGTACGATCACAAGGAATCCTGT
AATGGCCTGTACTATCAGGGCTCCTGTTACATCCTGCACTCTGACTATAAGAGCTTTGA
GGATGCCAAGGCCAACTGTGCCGCCGAGTCCTCTACCCTGCCAAATAAGAGCGACGTG
CTGACCACATGGCTGATCGACTATGTGGAGGATACCTGGGGCTCTGATGGCAACCCCAT
CACAAAGACCACATCTGATTACCAGGACAGCGACGTGAGCCAGGAGGTGCGGAAGTA
TTTCTGCACCGCTGCTGCTTCCATCCAGACCACAGTGAATACACTGAGCGAGAGAATC
AGCTCCAAACTGGAGCAGGAGGCCAACGCCTCTGCCCAGACCAAGTGTGACATCGAG
ATCGGCAACTTCTACATCAGGCAGAATCATGGATGTAATATCACCGTGAAGAACATGTG
CTCCGCCGATGCCGACGCCCAGCTGGACGCCGTGCTGAGCGCCGCCACTGAGACATAC
TCCGGCCTGACCCCCGAACAGAAAGCCTATGTGCCTGCTATGTTTACCGCCGCCCTGA
ACATTCAGACCAGCGTCAACACAGTGGTGCGCGATTTCGAAAATTATGTCAAACAGAC
TTGTAATTCTAGCGCCGTCGTCGATAACAAGCTGAAGATTCAGAATGTGATTATTGACG
AATGCTACGGAGCACCAGGCTCCCCAACCAACCTGGAGTTTATCAACACCGGCTCCTC
TAAGGGCAATTGTGCCATCAAGGCCCTGATGCAACTGACTACCAAAGCTACCACACAG
ATCGCACCAAGGCAGGTGGCAGGA
SEQ ID NO: 14 - the mRNA sequence encoding DAM (in which A1 and A2 are the same, both as shown in SEQ ID NO: 1) 
AGCACCACCCAGUACGACCACAAAGAGAGCUGCAACGGCCUGUACUACCAAGGCUC
UUGUUACAUCCUGCACAGCGACUAUAAGAGCUUCGAGGAUGCUAAGGCCAAUUGC
GCCGCCGAGAGCAGCACACUGCCUAACAAGUCCGACGUGCUGACCACCUGGCUGAU
CGACUACGUGGAAGAUACAUGGGGAAGCGACGGCAACCCCAUCACAAAAACCACAA
GCGAUUAUCAGGACAGCGACGUGUCUCAGGAGGUUAGAAAGUACUUUUGCACCGC
CGCCGCCUCUAUCCAGACCACAGUGAACACCCUGAGCGAGCGGAUCAGCUCCAAGC
UGGAGCAGGAGGCAAACGCAAGCGCCCAGACAAAGUGCGACAUCGAGAUCGGCAA
UUUCUACAUCAGACAGAACCACGGCUGCAAUAUCACCGUGAAGAAUAUGUGCUCC
GCCGACGCAGAUGCACAGCUGGAUGCCGUGCUGUCCGCCGCAACCGAGACAUACUC
 
UGGCCUGACCCCUGAGCAGAAGGCCUAUGUGCCAGCCAUGUUCACAGCCGCCCUGA
ACAUCCAGACCAGCGUGAAUACAGUGGUGCGGGACUUUGAGAACUACGUGAAGCA
GACCUGCAAUUCUAGCGCCGUGGUGGACAACAAGCUGAAGAUCCAGAAUGUGAUC
AUCGAUGAGUGUUAUGGAGCACCAGGCUCCCCUACCAACCUGGAGUUCAUCAAUAC
AGGCUCCUCUAAGGGCAACUGCGCCAUCAAGGCCCUGAUGCAGCUGACCACAAAGG
CCACCACACAGAUCGCACCUAGGCAGGUGGCAGGCAGCACCACACAGUACGAUCAC
AAGGAAUCCUGUAAUGGCCUGUACUAUCAGGGCUCCUGUUACAUCCUGCACUCUG
ACUAUAAGAGCUUUGAGGAUGCCAAGGCCAACUGUGCCGCCGAGUCCUCUACCCUG
CCAAAUAAGAGCGACGUGCUGACCACAUGGCUGAUCGACUAUGUGGAGGAUACCU
GGGGCUCUGAUGGCAACCCCAUCACAAAGACCACAUCUGAUUACCAGGACAGCGAC
GUGAGCCAGGAGGUGCGGAAGUAUUUCUGCACCGCUGCUGCUUCCAUCCAGACCAC
AGUGAAUACACUGAGCGAGAGAAUCAGCUCCAAACUGGAGCAGGAGGCCAACGCC
UCUGCCCAGACCAAGUGUGACAUCGAGAUCGGCAACUUCUACAUCAGGCAGAAUCA
UGGAUGUAAUAUCACCGUGAAGAACAUGUGCUCCGCCGAUGCCGACGCCCAGCUGG
ACGCCGUGCUGAGCGCCGCCACUGAGACAUACUCCGGCCUGACCCCCGAACAGAAA
GCCUAUGUGCCUGCUAUGUUUACCGCCGCCCUGAACAUUCAGACCAGCGUCAACAC
AGUGGUGCGCGAUUUCGAAAAUUAUGUCAAACAGACUUGUAAUUCUAGCGCCGUC
GUCGAUAACAAGCUGAAGAUUCAGAAUGUGAUUAUUGACGAAUGCUACGGAGCAC
CAGGCUCCCCAACCAACCUGGAGUUUAUCAACACCGGCUCCUCUAAGGGCAAUUGU
GCCAUCAAGGCCCUGAUGCAACUGACUACCAAAGCUACCACACAGAUCGCACCAAG
GCAGGUGGCAGGA
SEQ ID NO: 15 - the mRNA sequence encoding DAM (in which A1 and A2 are different) 
AGCACCACCCAGUACGACCACAAAGAGAGCUGCAACGGCCUGUACUACCAAGGCUC
UUGUUACAUCCUGCACAGCGACUAUAAGAGCUUCGAGGAUGCUAAGGCCAAUUGC
GCCGCCGAGAGCAGCACACUGCCUAACAAGUCCGACGUGCUGACCACCUGGCUGAU
CGACUACGUGGAAGAUACAUGGGGAAGCGACGGCAACCCCAUCACAAAAACCACAA
GCGAUUAUCAGGACAGCGACGUGUCUCAGGAGGUUAGAAAGUACUUUUGCACCGC
CGCCGCCUCUAUCCAGACCACAGUGAACACCCUGAGCGAGCGGAUCAGCUCCAAGC
UGGAGCAGGAGGCAAACGCAAGCGCCCAGACAAAGUGCGACAUCGAGAUCGGCAA
 
UUUCUACAUCAGACAGAACCACGGCUGCAAUAUCACCGUGAAGAAUAUGUGCUCC
GCCGACGCAGAUGCACAGCUGGAUGCCGUGCUGUCCGCCGCAACCGAGACAUACUC
UGGCCUGACCCCUGAGCAGAAGGCCUAUGUGCCAGCCAUGUUCACAGCCGCCCUGA
ACAUCCAGACCAGCGUGAAUACAGUGGUGCGGGACUUUGAGAACUACGUGAAGCA
GACCUGCAAUUCUAGCGCCGUGGUGGACAACAAGCUGAAGAUCCAGAAUGUGAUC
AUCGAUGAGUGUUAUGGAGCACCAGGCUCCCCUACCAACCUGGAGUUCAUCAAUAC
AGGCUCCUCUAAGGGCAACUGCGCCAUCAAGGCCCUGAUGCAGCUGACCACAAAGG
CCACCACACAGAUCGCACCUAGGCAGGUGGCAGGCUCCGCCAAUAAGGCAGCCAUC
ACCGACUCUGCCGUGGCAGUGGCAGCAGCCAGCUCCACACACCGCAAGGUGGUGUC
UAGCACCACACAGUACGAUCACAAGGAAUCCUGUAAUGGCCUGUACUAUCAGGGC
UCCUGUUACAUCCUGCACUCUGACUAUAAGAGCUUUGAGGAUGCCAAGGCCAACU
GUGCCGCCGAGUCCUCUACCCUGCCAAAUAAGAGCGACGUGCUGACCACAUGGCUG
AUCGACUAUGUGGAGGAUACCUGGGGCUCUGAUGGCAACCCCAUCACAAAGACCAC
AUCUGAUUACCAGGACAGCGACGUGAGCCAGGAGGUGCGGAAGUAUUUCUGCACC
GCUGCUGCUUCCAUCCAGACCACAGUGAAUACACUGAGCGAGAGAAUCAGCUCCAA
ACUGGAGCAGGAGGCCAACGCCUCUGCCCAGACCAAGUGUGACAUCGAGAUCGGCA
ACUUCUACAUCAGGCAGAAUCAUGGAUGUAAUAUCACCGUGAAGAACAUGUGCUC
CGCCGAUGCCGACGCCCAGCUGGACGCCGUGCUGAGCGCCGCCACUGAGACAUACU
CCGGCCUGACCCCCGAACAGAAAGCCUAUGUGCCUGCUAUGUUUACCGCCGCCCUG
AACAUUCAGACCAGCGUCAACACAGUGGUGCGCGAUUUCGAAAAUUAUGUCAAAC
AGACUUGUAAUUCUAGCGCCGUCGUCGAUAACAAGCUGAAGAUUCAGAAUGUGAU
UAUUGACGAAUGCUACGGAGCACCAGGCUCCCCAACCAACCUGGAGUUUAUCAACA
CCGGCUCCUCUAAGGGCAAUUGUGCCAUCAAGGCCCUGAUGCAACUGACUACCAAA
GCUACCACACAGAUCGCACCAAGGCAGGUGGCAGGA
SEQ ID NO: 16 - 5'UTR sequence
   GGGAAATAAGAGAGAAAAGAAGAGTAAGAAGAAATATAAGAGCCACC
SEQ ID NO: 17 - signal peptide sequence
   METDTLLLWVLLLWVPGSTG
SEQ ID NO: 18 - coding sequence of signal peptide 
ATGGAAACAGACACCCTGCTGCTGTGGGTCCTGCTGCTCTGGGTGCCTGGCAGCACCG
GC
SEQ ID NO: 19 - 3'UTR sequence

TGATAATAGGCTGGAGCCTCGGTGGCCATGCTTCTTGCCCCTTGGGCCTCCCCCCAGCC
CCTCCTCCCCTTCCTGCACCCGTACCCCCGTGGTCTTTGAATAAAGTCTGA

## Claims

1. A polynucleotide which encodes a multi-immunogen chimeric or mixed poxvirus antigen, wherein the chimeric or mixed poxvirus antigen contains:
an immunogen I: a monkeypox virus A35R protein or its antigenic fragment, or an amino acid sequence having at least 90%, 92%, 95%, 96%, 97%, 98%, or 99% identity thereto and having the same or substantially the same immunogenicity therewith; and
an immunogen II: a monkeypox virus M1R protein or its antigenic fragment, or an amino acid sequence having at least 90%, 92%, 95%, 96%, 97%, 98%, or 99% identity thereto and having the same or substantially the same immunogenicity therewith.

2. The polynucleotide according to claim 2, wherein the antigenic fragment of the A35R protein is an extracellular fragment of the protein or a part thereof, preferably a peptide fragment having an amino acid sequence as shown in SEQ ID NO: 1, or an amino acid sequence consisting of the amino acid sequence as shown in SEQ ID NO: 1 plus from 1 to 30 amino acids extending therefrom toward the N-terminus of the A35R protein, which sequence is preferably as shown in SEQ ID NO: 2;
and/or, the antigenic fragment of the M1R protein is an extracellular fragment of the protein or a part thereof, preferably a peptide fragment having an amino acid sequence as shown in SEQ ID NO: 3.

3. The polynucleotide according to claim 1 or 2, wherein the polynucleotide encodes a multi-immunogen mixed poxvirus antigen, which is a mixture of the immunogens I and II;
wherein, preferably, the immunogen I has an amino acid sequence as shown in SEQ ID NO: 1 or SEQ ID NO: 2, or an amino acid sequence that is derived from the amino acid sequence as shown in SEQ ID NO: 1 or SEQ ID NO: 2 by substitution, deletion, or addition of one or more amino acids, and exhibits the same or substantially the same immunogenicity therewith;
and/or, the immunogen II has an amino acid sequence as shown in SEQ ID NO: 3, or an amino acid sequence that is derived from the amino acid sequence as shown in SEQ ID NO: 3 by substitution, deletion, or addition of one or more amino acids, and exhibits the same or substantially the same immunogenicity therewith;
preferably, in the mixture, the mass ratio of the immunogens I and II is 1:1.

4. The polynucleotide according to claim 3, wherein the polynucleotide is a group of polynucleotides, comprising:
I) a DNA molecule with a sequence as shown in SEQ ID NO: 4 or 5, and/or its corresponding mRNA molecule, which sequence is as shown in SEQ ID NO: 6 or 7 respectively; and
II) a DNA molecule with a sequence as shown in SEQ ID NO: 8, and/or its corresponding mRNA molecule, which sequence is as shown in SEQ ID NO: 9.

5. The polynucleotide according to claim 1 or 2, wherein the polynucleotide encodes a multi-immunogen chimeric poxvirus antigen, which is a single chain formed by one or more immunogens I and II in a series mode;
preferably, the chimeric antigen has a structure as shown in Formula (I):
A1-C₁-M1-C₂-A2-C₃-M2 (I)
in the Formula (I):
A1 represents the monkeypox virus A35R protein or its antigenic fragment I, or an amino acid sequence having at least 90%, 92%, 95%, 96%, 97%, 98%, or 99% identity thereto and having the same or substantially the same immunogenicity therewith,
A2 represents the monkeypox virus A35R protein or its antigenic fragment II, or an amino acid sequence having at least 90%, 92%, 95%, 96%, 97%, 98%, or 99% identity thereto and having the same or substantially the same immunogenicity therewith,
M1 represents the monkeypox virus M1R protein or its antigenic fragment I, or an amino acid sequence having at least 90%, 92%, 95%, 96%, 97%, 98%, or 99% identity thereto and having the same or substantially the same immunogenicity therewith,
M2 represents the monkeypox virus M1R protein or its antigenic fragment II, or an amino acid sequence having at least 90%, 92%, 95%, 96%, 97%, 98%, or 99% identity thereto and having the same or substantially the same immunogenicity therewith;
C₁, C₂, and C₃ are each independently null, or a linker sequence (GGGGS)n, wherein n is any integer between 1 and 10; and,
wherein,
A1 and A2 are the same or different,
M1 and M2 are the same or different.

6. The polynucleotide according to claim 5, wherein the antigenic fragment I or II of the A35R protein is an extracellular fragment of the protein or a part thereof;
and/or, the antigenic fragment I or II of the M1R protein is an extracellular fragment of the protein or a part thereof.

7. The polynucleotide according to claim 5 or 6, wherein A1 represents the amino acid sequence as shown in SEQ ID NO: 1, or an amino acid sequence that is derived from the amino acid sequence as shown in SEQ ID NO: 1 by substitution, deletion, or addition of one or more amino acids, and exhibits the same or substantially the same immunogenicity therewith;
and/or, A2 represents the amino acid sequence as shown in SEQ ID NO: 1, or an amino acid sequence consisting of the amino acid sequence as shown in SEQ ID NO: 1 plus from 1 to 30 amino acids extending therefrom toward the N-terminus of the A35R protein, which sequence is preferably as shown in SEQ ID NO: 2, or an amino acid sequence that is derived from any of the above amino acid sequences by substitution, deletion, or addition of one or more amino acids, and exhibits the same or substantially the same immunogenicity therewith;
and/or, M1 and M2 are the same and represent the amino acid sequence as shown in SEQ ID NO: 3, or an amino acid sequence that is derived from the amino acid sequence as shown in SEQ ID NO: 3 by substitution, deletion, or addition of one or more amino acids, and exhibits the same or substantially the same immunogenicity therewith;
preferably, A1 represents the amino acid sequence as shown in SEQ ID NO: 1, A2 represents the amino acid sequence as shown in SEQ ID NO: 1 or SEQ ID NO: 2, M1 and M2 are the same and represent the amino acid sequence as shown in SEQ ID NO: 3;
preferably, C1, C2, and C3 are all null;
further preferably, the chimeric antigen contains an amino acid sequence as shown in SEQ ID NO: 10 or 11;
optionally, the chimeric antigen is of a single-chain dimer structure.

8. The polynucleotide according to claim 7, wherein the polynucleotide is a DNA molecule and/or its corresponding mRNA molecule;
preferably, the DNA molecule has a DNA sequence as shown in SEQ ID NO: 12 or 13, and its corresponding mRNA molecule has an mRNA sequence as shown in SEQ ID NO: 14 or 15.

9. A nucleic acid construct, which comprises the polynucleotide according to any one of claims 1-8 and optionally, at least one expression regulatory element operatively linked to the polynucleotide.

10. An expression vector, which comprises the nucleic acid construct according to claim 9.

11. A host cell, into which the polynucleotide according to any one of claims 1-8, the nucleic acid construct according to claim 9, or the expression vector according to claim 10 is transformed or transfected.

12. Use of the polynucleotide according to any one of claims 1-8, the nucleic acid construct according to claim 9, the expression vector according to claim 10, or the host cell according to claim **11** in the preparation of a medicament for preventing and/or treating poxvirus infections; preferably, the poxvirus is selected from: monkeypox virus, smallpox virus, cowpox virus, and/or vaccinia virus;
optionally, the medicament is a vaccine, preferably an mRNA vaccine;
optionally, the vaccine is in a form of a nasal spray, an oral preparation, a suppository, or a parenteral preparation;
preferably, the nasal spray is selected from an aerosol, a spray, and a dry powder inhaler;
preferably, the oral preparation is selected from a tablet, a powder, a pill, granules, a soft/hard capsule, a film-coated agent, and a paste; further preferably, the tablet is a sublingual tablet; further preferably, the granules are fine granules; further preferably, the powder is a pulvis; and further preferably, the pill is a pilule;
preferably, the parenteral preparation is a transdermal preparation, an ointment, a plaster, a topical liquid preparation, or an injectable preparation; and further preferably, the injectable preparation is a preparation for infusion.

13. A nucleic acid vaccine or immunogenic composition, which comprises the polynucleotide according to any one of claims 1-8, the nucleic acid construct according to claim 9, the expression vector according to claim 10, or the host cell according to claim 11, as well as a physiologically acceptable vehicle, adjuvant, excipient, carrier, and/or diluent.

14. The nucleic acid vaccine or immunogenic composition according to claim 13, which is a DNA vaccine against a poxvirus, comprising:
(i) an eukaryotic expression vector; and
(ii) a DNA sequence of the polynucleotide according to any one of claims 1-8, which is constructed into the eukaryotic expression vector;
preferably, the eukaryotic expression vector is selected from pGX0001, pVAX1, pCAGGS, and pcDNA series vectors.

15. The nucleic acid vaccine or immunogenic composition according to claim 13, which is an mRNA vaccine against a poxvirus, comprising:
(I) an mRNA sequence of the polynucleotide according to any one of claims 1-8; and
(II) lipid nanoparticles.

16. The nucleic acid vaccine or immunogenic composition according to claim 13, which is a poxvirus-virus vector vaccine, comprising:
(1) a viral backbone vector; and
(2) a DNA sequence of the polynucleotide according to any one of claims 1-8, which is constructed into the viral backbone vector;
preferably, the viral backbone vector is selected from one or more of the following viral vectors: an adenovirus vector, a poxvirus vector, an influenza virus vector, and an adeno-associated virus vector.

17. The nucleic acid vaccine or immunogenic composition according to any one of claims 13-16, wherein the nucleic acid vaccine or immunogenic composition is in a form of a nasal spray, an oral preparation, a suppository, or a parenteral preparation;
preferably, the nasal spray is selected from an aerosol, a spray, and a dry powder inhaler;
preferably, the oral preparation is selected from a tablet, a powder, a pill, granules, a soft/hard capsule, a film-coated agent, and a paste;
further preferably, the tablet is a sublingual tablet;
further preferably, the granules are fine granules;
further preferably, the powder is a pulvis;
further preferably, the pill is a pilule;
preferably, the parenteral preparation is a transdermal preparation, an ointment, a plaster, a topical liquid preparation, or an injectable preparation; and further preferably, the injectable preparation is a preparation for infusion.

18. A kit, which comprises the polynucleotide according to any one of claims 1-8, the nucleic acid construct according to claim 9, the expression vector according to claim 10, the host cell according to claim 11, or the nucleic acid vaccine or immunogenic composition according to any one of claims 13-17.
